# EUROPEAN PATENT APPLICATION

(11) **EP 2 338 464 A2**
(43) Date of publication of application: **29.06.2011**
(21) Application number: 10169973.4
(22) Date of filing: 19.07.2010
(51) Int. Cl.: A61K 8/19, A61K 8/21, A61Q 11/00

(54) **Oral care compositions which comprise stannous and potassium salts**

(30) Priority: 27.07.2009 EP 09166510
(71) Applicant: The Procter & Gamble Company, Cincinnati, OH 45202 (US)
(72) Inventor: Strand, Ross, Bracknell, Berkshire RG42 3RZ (GB)
(74) Representative: Clemo, Nicholas Graham

(57) **Abstract**

The present invention relates to a single phase oral care composition comprising:
a. a stannous salt delivering a stannous ion;
b. a potassium salt delivering a potassium ion;
c. a chelant;
d. a fluoride ion source;
e. less than 0.01% of an alkyl sulphate or an alkyl ethoxylate sulphate; and
f. a surfactant selected from a cationic, an amphoteric surfactant, a nonionic surfactant or mixtures thereof;
wherein the oral care composition provides a soluble fluoride ion level of greater than 50% of the total fluoride ion.

The composition of the invention has been found to allow prolonged contact between stannous ion and nitrate ion in a single dentifrice without toxic effects or insoluble products. The invention further provides for the maintenance of an efficacious fluoride ion level.

## Description

### FIELD OF THE INVENTION

The present invention relates to oral care compositions comprising both tin (II) and potassium ions.

### BACKGROUND OF THE INVENTION

Dentinal hypersensitivity is defined as acute, temporary, localised tooth pain in response to changes in temperature, pressure or chemistry. Exposure of the dentine, often due to recession of the gums, or loss of enamel, frequently leads to hypersensitivity. Dentinal tubules which are open to the surface correlate with hypersensitivity. Dentinal tubules lead from the pulp to the cementum. When the surface cementum of the tooth root is eroded, or exposed by periodontal disease, the tubules become exposed to the external environment and provide a pathway for the passage of fluid to the pulpal nerves.

"Nerve desensitising agents" can reduce the excitability of a nerve in a sensitive tooth by altering the chemical environment. It is known that potassium salts are effective in this way in the treatment of dentinal hypersensitivity. US3863006 discloses that potassium salts such as potassium nitrate, when incorporated in toothpastes, desensitise the teeth. It is believed that an elevated extra cellular potassium concentration close to the pulpal nerves underlying sensitive dentin is responsible for the desensitising effect of oral care products which contain potassium salts.

An alternative way to treat hypersensitivity is to use an agent which partially or fully occludes the dentinal tubules. Tin (II) (stannous) ions, provided in oral compositions by stannous fluoride and/or other stannous salts, have long been valued for the multiple benefits that they can afford, including antimicrobial effects, control of breath malodour, control of dental plaque growth and metabolism, reduced gingivitis, decreased progression to periodontal disease, reduced coronal and root dental caries and erosion and reductions in dentinal hypersensitivity. Stannous salts are known to be efficacious in the reduction of dentinal hypersensitivity via this method as disclosed in US6592853 amongst others. Stannous is known in the art to occlude the dentin tubules and thus dramatically reduce fluid flow within the tubules which stimulate pain.

There are several disclosures of two component desensitising dentifrice in the prior art where the first component contains a potassium salt and the second component contains a stannous salt.

The two components are generally maintained separately from each other until dispensed for application to teeth. Such compositions are disclosed in Colgate patents US5780015, US5693314, US5932192, US5843409, US6464963. The partial occlusion of the tubules by stannous ions is believed to increase the flux of potassium ions into the tooth as the inward diffuse flux is less dependent on the tubule radius than the outward fluid flow. It is disclosed in the art that attempts to include mixtures of desensitising agents such as stannous salts and potassium salts in a single desensitising dual composition have been found to be of limited effect as a means for delivering efficacious amounts of both ingredients to the teeth. US6464963 describes how insoluble stannic salts and stannous compounds are formed during storage. The present inventors overcome this problem via the addition of a chelant.

US5843409 discloses that prolonged contact between stannous ion and nitrate ion in a single dentifrice results in a reaction of these ions causing a conversion of nitrate into potentially toxic materials. It is believed from chemical first principles that the pre-cursor to any such products would be production of nitrites. Reducing agents such as stannous can convert nitrates into nitrites. Under acidic conditions the nitrite forms nitrous acid which is protonated and forms the nitrosonium cation. This can react with amines in the oral cavity to produce the toxic substance, nitrosamine. Careful stabilisation of the stannous via chelating agents can prevent this from happening. The present inventors have surprisingly found that there is no need for dual component toothpastes with dual containers to keep the stannous ion and potassium nitrate separate from each other. In aqueous models of nitrate and stannous containing dentifrices there were no signs of formation of nitrite over a wide pH range.

As described in EP1040819, sodium alkylsulphate surfactants, for example sodium lauryl sulphate (SLS), are generally not compatible with compounds that contain potassium because an insoluble potassium alkylsulphate precipitate forms when the sodium alkylsulphate is combined with a potassium salt. Although the combination of potassium and SLS is known to be unfavourable, many marketed products still use this formulation. This is generally managed by maintaining a low ionic strength within these formulations. However, the introduction of a stannous salt with the associated chelants required to prevent the formation of insoluble products, such as in the composition of the present invention, drives an increase in ionic strength and results in increased precipitate levels. The present inventors have discovered that excluding SLS from the present oral care composition overcomes this problem. In addition, the exclusion of SLS from the present composition allows an efficacious level of fluoride to be maintained as compared with the compositions which utilise SLS. Without wishing to be bound by theory, it is believed that eliminating the potential potassium alkyl sulphate precipitate changes the overall solubilising capacity of the composition and thus allows an efficacious level of fluoride to be maintained. Fluoride ions are well known in the art to provide anti-caries benefits. Fluoride enhances remineralisation, acts anti-bacterially and strengthens enamel. It is thus desirable to treat sensitivity as well as caries in a single formulation.

### SUMMARY OF THE INVENTION

The present invention relates to a single phase oral care composition comprising:
a. a stannous salt delivering a stannous ion;
b. a potassium salt delivering a potassium ion;
c. a chelant;
d. a fluoride ion source;
e. less than 0.01 % of an alkyl sulphate or an alkyl ethoxylate sulphate; and
f. a surfactant selected from a cationic, an amphoteric surfactant, a nonionic surfactant or mixtures thereof;
wherein the oral care composition provides a soluble fluoride ion level of greater than 50% of the total fluoride ion.

The composition of the invention has been found to allow prolonged contact between stannous ion and nitrate ion in a single phase dentifrice without toxic effects or insoluble products. The invention further provides for the maintenance of an efficacious fluoride ion level.

### DETAILED DESCRIPTION OF THE INVENTION

Unless specified otherwise, all percentages and ratios herein are by weight of the total composition and all measurements are made at 25°C.

The present invention relates to a single phase oral care composition. The composition can be in the form of a mouth spray, mouthwash or a toothpaste or gel. Preferably the composition is in the form of a toothpaste or tooth gel suitable for use in brushing teeth.

The oral care compositions herein are single phase, by which is meant that all of the ingredients of the composition are containable within in a single compartment of a container and no further mixing is required before use.

### Stannous ions

A first ingredient of the present oral care composition is a source of tin (II) (stannous) ions which preferably provides from 0.05% to 1.20% (500 to 12000 ppm) stannous ions, more preferably from 0.10% to 0.80% (1000 to 8000 ppm) stannous ions and even more preferably from 0.25% to 0.70% (2500 to 7000 ppm) stannous ions. Suitable stannous sources include stannous fluoride, stannous chloride, stannous acetate, stannous gluconate, stannous oxalate, stannous sulfate, stannous lactate and stannous tartrate. Especially preferred sources of tin (II) ions are stannous chloride, stannous fluoride, stannous gluconate and mixtures thereof due to their establishment as clinically proven salts to deliver stannous ions.

### Potassium ions

A second ingredient of the present oral care composition is a source of potassium ions which preferably provides from 0.90% to 4.0% (9000 to 40000 ppm) potassium ions, more preferably from 1.50% to 3.60% (15000 to 36000 ppm) potassium ions and even more preferably from 1.90% to 2.50% (19000 to 25000 ppm) potassium ions. Suitable potassium sources include potassium nitrate, potassium gluconate, potassium citrate, potassium chloride, potassium tartrate, potassium bicarbonate, potassium oxalate, and mixtures thereof. Potassium nitrate, potassium gluconate, potassium citrate, potassium chloride and mixtures thereof are preferred due to their establishment as clinically proven salts to deliver potassium ions. In a further embodiment, potassium hydroxide can be used a potassium source.

### Chelants

The oral composition of the invention comprises one or more chelants, also known as chelating agents. The term "chelant", as used herein means a bi- or multidentate ligand having at least two groups capable of binding to stannous ions and preferably other divalent or polyvalent metal ions and which, at least as part of a chelant mixture, is capable of solubilising the stannous ions and other optional metal ions within the oral composition. Groups capable of binding to stannous and other metal ions include carboxyl, hydroxl and amine groups. Typically, those chelants useful herein will also form water soluble stable complexes with the stannous ions.

Suitable chelants herein include C₂ - C₆ dicarboxylic and tricarboxylic acids, such as succinic acid, malic acid, tartaric acid and citric acid; C₃ - C₆ monocarboxylic acids substituted with hydroxyl, such as gluconic acid; picolinic acid; amino acids such as glycine; phytic acid, salts thereof and mixtures thereof. The chelant can also be a polymer or copolymer in which the chelating ligands are on the same or adjacent monomer. Preferred chelant polymers are polyacids selected from the group consisting of a homopolymer of a monomer, a copolymer of two or more different monomers, and a combination thereof wherein the monomer or at least one of the two or more different monomers is selected from the group consisting of acrylic acid, methacrylic acid, itaconic acid, maleic acid, glutaconic acid, aconitic acid, citraconic acid, mesaconic acid, fumaric acid and tiglic acid. Particularly preferred is a methylvinylether/maleic acid (PVM/MA) copolymer.

Also suitable as chelants are polyphosphates such as tripolyphosphates. Longer chain linear polyphosphates, though good chelants, are susceptible to hydrolysis in aqueous compositions. Upon hydrolysis they form orthophosphates which form insoluble zinc complexes. They are therefore preferably used in anhydrous compositions.

Some materials, orthophosphate in particular, might be considered to be chelants in that they are bi- or multidentate ligands having at least two groups capable of binding to the divalent metal ions but nevertheless form insoluble zinc salts and are therefore not useful chelants for compositions which comprise zinc ions.

Phytate is a preferred chelant herein because it also provides stain removal benefits. However, because stannous phytate is partially soluble it is preferably not used as the sole chelant and is preferably used in combination with the organic acids described in this section. Preferred organic acid chelants herein comprise citrate, malate, tartrate, gluconate, succinate, lactate, malonate, maleate, and mixtures thereof, whether added in their free acid or salt forms.

The chelants in the composition will preferably be in range 0.1 % to 10% of the composition to stabilize the stannous ions.

For chelants with a molecular weight of less than 1000, the molar ratio of the chelant(s) used to the stannous ion delivered from the stannous salt is preferably at least 0.70:1, more preferably at least 0.8:1 and preferably 0.70:1 to 20:1. If other divalent metal ions, such as zinc, are added to the composition then the chelants should preferably be increased to a ratio of at least 0.70:1 of chelants to total metal ions. The molar ratio of chelants to divalent metal ions is the total number of moles of chelant(s) divided by the total number of moles of metal ions.

As a ratio of percentage weight of the chelant(s) to the stannous ion delivered from the stannous salt, particularly where one or more of the chelants has a molecular weight of greater than 1000, the composition will preferably have a ratio of chelant to stannous ion of at least 2:1, more preferably at least 5:1 and preferably 2:1 to 10:1. If other divalent metal ions, such as zinc, are added to the composition then the chelants should preferably be increased to maintain a ratio of at least 2:1 of chelants to total metal ions.

### Surfactant

The compositions of the present invention will include a cationic, an amphoteric, a nonionic surfactant or mixtures thereof.

It is known that the anionic alkyl sulphate surfactants, such as sodium lauryl sulphate (SLS), and alkyl ethoxylate sulphates precipitate in the presence of potassium ions. Although the combination of potassium and SLS is known to be unfavourable, many marketed products still use this formulation; generally managed by maintaining a low ionic strength. However, the present inventors have discovered that excluding alkyl sulphates, in particular SLS, from a high ionic strength composition, such as that of the present invention, solves this problem and also allows an efficacious level of fluoride to be maintained as compared with the compositions which utilise alkyl sulphates such as SLS. Without wishing to be bound by theory, it is believed that eliminating the potential potassium alkyl sulphate precipitate changes the overall solubilising capacity of the composition and thus allows an efficacious level of fluoride to be maintained. Therefore the present oral care compositions comprise less than 0.01 % of an alkyl sulphate or an alkyl ethoxylate sulphate, preferably less than 0.01% of an alkyl sulphate and even more preferably less than 0.01% SLS. The total level of alkyl sulphate and alkyl ethoxylate sulphate is less than 0.01 %.

Cationic surfactants can also be used though care needs to be taken over their compatibility with other ingredients. They would typically be used at levels similar to those of the additional anionic surfactants. Cationic surfactants useful in the present invention include derivatives of aliphatic quaternary ammonium compounds having one long alkyl chain containing from 8 to 18 carbon atoms such as lauryl trimethylammonium chloride; cetyl pyridinium chloride; cetyl trimethylammonium bromide; di-isobutylphenoxyethyl-dimethylbenzylammonium chloride; cetyl pyridinium fluoride; benzalkonium chloride; cetrimonium chloride; etc. Some of these cationic surfactants are also useful as anti-microbial agents. Some nonionic surfactants may be useful at substantially higher levels, such as up to 20% if it is desired to use them to form a ringing gel. Examples of suitable nonionic surfactants include the poloxamers, polyethylene oxide condensates of alkyl phenols, long chain tertiary amine oxides, long chain tertiary phosphine oxides, long chain dialkyl sulfoxides, cocamide MEA, coamide DEA and mixtures of such materials.

Preferred surfactants are amphoteric surfactants which would typically be used in an amount from 0.1% to 2.5%, preferably from 0.3% to 2.5% and most preferably from 0.5% to 2.0% by weight. Useful surfactants include cocamidopropyl hydroxysultaine; sodium cocoamphoacetate; disodium cocoamphodiacetate; dodecyl betaine; cocoamidoethyl betaine; cocamidopropyl betaine; cocamidopropyl betaine; lauramidopropyl betaine; lauryl betaine and mixtures there of. Especially preferred are cocoamidoethyl betaine; cocamidopropyl betaine; cocamidopropyl betaine; lauramidopropyl betaine; lauryl betaine and mixtures there of.

### Fluoride ions

The oral care composition comprises a source fluoride ions which will provide free fluoride ions in an oral care composition. It is common to have a water-soluble fluoride compound present in dentifrices and other oral compositions in an amount sufficient to give a fluoride ion concentration sufficient to provide anticaries effectiveness. The oral composition herein comprises a fluoride ion source sufficient to provide from 0.01% to 0.35% (100 to 3500 ppm) fluoride ions, preferably from 0.05% to 0.25% (500 to 2500 ppm) fluoride ions. The composition of the present invention has a soluble fluoride ion level of greater than 50% of the total fluoride ion, preferably greater than 75%. The exclusion of SLS facilitates this efficacious level. A wide variety of fluoride ion-yielding materials can be employed as sources of soluble or sparingly soluble fluoride ions in the present compositions. Representative fluoride ion sources include: stannous fluoride, sodium fluoride, potassium fluoride, indium fluoride, amine fluoride, and many others. Preferred sources of fluoride ion are stannous fluoride and sodium fluoride, as well as mixtures thereof. Monofluorophosphate (MFP), commonly used in oral care compositions, does not provide free fluoride ions in water, in contrast with the fluoride sources mentioned above. MFP provides ions of monofluorophosphate (FPO₃²⁻) when dissolved in water. This is broken down by enzymes (phosphatases) to provide free fluoride ions (F-) over time in-situ. It does not therefore provide a source of free fluoride ions in the oral care composition of the present invention.

Soluble fluoride within oral compositions of the present invention can be measured as follows.

Into a 50ml centrifuge tube, weigh 1g±0.01g composition and 9g±0.01g 10% deionised water. Add 6 glass balls and cap. Vortex for 2 minutes, then centrifuge for 10 mins at 15000 rpm at 37°C. Weigh 2g±0.01g supernatant into a beaker, add 18g±0.01g EDTA/TRIS buffer. Stir well to mix.

Reference solutions are made as followed.

### Fluoride stock solution 500mg/L F⁻

Weigh 1.105g+/0.001g sodium fluoride into a 1L volumetric flask. Dissolve in deionised water and dilute to volume.
Weigh the amounts of EDTA and TRIS into a bottle with the specified water and dissolve.

| Volume water (ml) | Weight EDTA (g) | Weight TRIS (g) | Volume Triton (ml) | Volumetric Flask (L) |
|---|---|---|---|---|
| 1750 | 148.90 | 48.46 | 10 | 2 |

Adjust the pH to 8.0+/- 0.05 with 50% sodium hydroxide and transfer to appropriate flask and dilute to volume. Add Triton X-100 then decant back to bottle for storage. The solution is stable for 12 months.

### 5mg/L fluoride solution

Weigh 1g+/- 0.05g stock solution into 10mml plastic bottle, add 9g +/-0.1g deionised water, then 90g+/-0.1g EDTA/TRIS buffer. Cap and mix well.

### 25mg/L fluoride solution

Weigh 5g+/- 0.05g stock solution into 10mml plastic bottle, add 5g +/-0.1g deionised water, then 90g+/-0.1g EDTA/TRIS buffer. Cap and mix well.

### 50mg/L fluoride solution

Weigh 10g+/- 0.1g stock solution into 10mml plastic bottle, then add 90g+/-0.1g EDTA/TRIS buffer. Cap and mix well.

Using the above reference solutions of 5.0, 25.0 and 50.0 mg/L solutions of fluoride in EDTA/TRIS buffer the amount of fluoride can be measured using any suitable ion meter and ion selective electrode. The soluble fluoride level is calculated from the electrode reading, taking into account the dilution factor of both the sample and the references. The method can be adjusted accordingly for other fluoride salts.

In preferred compositions of the present invention the level of soluble fluoride ion is in the range from 25 to 930 ppm, preferably from 130 to 660 ppm, as measured when the sample has been diluted 1:3 with deionised water.

### Zinc ions

Zinc ions may advantageously be included in oral compositions. Combining zinc ions with stannous ions can give a broader spectrum of anti-microbial activity. The present composition may include a source of zinc ions sufficient to provide from 0.1 to 1.5%, preferably from 0.1 to 1%, more preferably from 0.15 to 0.5% zinc ions by weight of the composition. Insoluble or sparingly soluble zinc compounds, such as zinc oxide or zinc carbonate, can be used as the zinc source. Preferred zinc sources however are soluble zinc sources such as zinc chloride or zinc sulphate. More preferred zinc sources are those where the zinc is already combined with a suitable chelating agent in the form of a salt or other complex, such as zinc citrate, zinc gluconate, zinc lactate and zinc glycinate. Especially preferred sources of zinc ions are zinc citrate, zinc gluconate, zinc lactate and mixtures thereof.

The preferred pH range of the present composition, to avoid the precipitation of stannous, is less than 7.5, preferably less than 7 and more preferably less than 6.5, such as from 4.5 to 7.5, more preferably 5 to 7 and even more preferably 5.5 to 6.5. The pH of the oral care composition is preferably no lower than 4.5 for safety reasons. The pH of a dentifrice composition is measured from a 3:1 aqueous slurry of the dentifrice, i.e., 3 parts water to 1 part dentifrice.

### Water

The term "orally acceptable carrier" as used means a liquid or semi-solid vehicle such as a paste or a gel for containing the active ingredients of the present invention and delivering them to the oral cavity. Water is commonly used as a carrier material in oral compositions. It is useful as a processing aid, is benign to the mouth and it assists in quick foaming of toothpastes. Water may be added as an ingredient in its own right or it may be present as a carrier in other common raw materials such as sorbitol. The term 'total water' as used herein means the total amount of water present in the composition, whether added separately or as a solvent or carrier for other raw materials but excluding that which may be present as water of crystallisation in certain inorganic salts. Preferred dentifrice compositions herein are aqueous compositions comprising from 20% to 65%, preferably from 30% to 55%, more preferably from 40% to 50% total water. The carrier can also include other conventional additives in oral care compositions such as desensitizing agents, teeth whitening agents such as peroxide sources, herbal agents, buffers, anti-staining agents, thickening materials, humectants, surfactants, a flavour system, sweetening agents, and colouring agents.

### Other ingredients

The present oral care composition can comprise the usual and conventional ancillary components as more fully described hereinafter.

Dental abrasives are useful in oral compositions such as tooth pastes and gels for their ability to remove surface stain and pellicle and for polishing the teeth. A dental abrasive is a highly preferred ingredient of the present composition. Dental abrasives useful in the present oral composition of the subject invention include many different materials. The material selected must be one which is compatible with the composition of interest and does not excessively abrade dentin. Suitable abrasives include, for example, silicas including gels and precipitates, insoluble sodium polymetaphosphate, hydrated alumina, and resinous abrasive materials such as particulate condensation products of urea and formaldehyde. Another class of abrasives for use in the present compositions is particulate thermo-setting polymerized resins, as described in U.S. Pat. No. 3,070,510. Suitable resins include, for example, melamines, phenolics, ureas, melamine-ureas, melamine-formaldehydes, urea-formaldehyde, melamine-urea-formaldehydes, cross-linked epoxides, and cross-linked polyesters.

Silica dental abrasives of various types are preferred herein because of their unique benefits of exceptional dental cleaning and polishing performance without unduly abrading tooth enamel or dentine. Silica abrasive polishing materials herein, as well as other abrasives, generally have an average particle size ranging from 0.1 to 30 µm, and preferably from 5 to 15 µm. The abrasive can be precipitated silica or silica gels such as the silica xerogels described in U.S. Patent Nos. 3,538,230 and 3,862,307. Examples include the silica xerogels marketed under the trade name "Syloid" by the W.R. Grace & Company, Davison Chemical Division and precipitated silica materials such as those marketed by the J. M. Huber Corporation under the trade name, Zeodent®, particularly the silicas carrying the designation Zeodent^{®} 119, Zeodent^{®} 118, Zeodent^{®} 109 and Zeodent^{®} 129. The types of silica dental abrasives useful in the toothpastes of the present invention are described in more detail in U.S. Patent Nos. 4,340,583, 5,603,920, 5,589,160, 5,658,553, 5,651,958 and 6,740,311.

Mixtures of abrasives can be used, such as mixtures of the various grades of Zeodent® silica abrasives listed above. The total amount of abrasive in dentifrice compositions of the present invention typically ranges from 6% to 50% by weight of the composition. Dental solution, mouth spray, mouthwash and non-abrasive gel compositions of the subject invention typically contain little or no abrasive.

An optional but preferred component of the compositions herein is a humectant. The humectant serves to keep the dentifrice from hardening upon exposure to air, to give a moist feel to the mouth, and, for particular humectants, to impart a desirable sweetness of flavour. The humectant, on a pure humectant basis, generally comprises from 5% to 70%, preferably from 15% to 45%, by weight of the composition. Suitable humectants include edible polyhydric alcohols such as glycerin, sorbitol, xylitol, butylene glycol, polyethylene glycol, and propylene glycol, especially sorbitol and glycerin.

In preparing tooth pastes or gels, it is often necessary to add a thickening agent or binder to provide a desirable consistency of the composition, to provide desirable active release characteristics upon use, to provide shelf stability, and to provide stability of the composition, etc. Thickening agents can include carboxyvinyl polymers, carrageenan, nonionic cellulose derivatives such as hydroxyethyl cellulose (HEC), and water soluble salts of cellulose derivatives such as sodium carboxymethylcellulose (NaCMC). Natural gums such as gum karaya, xanthan gum, gum arabic, and gum tragacanth can also be used herein. Suitable thickening agent levels can range from 0.1 to 5%, and higher if necessary.

Organic antimicrobial agents may also be employed. Included among such agents are water insoluble non-cationic antimicrobial agents such as halogenated diphenyl ethers, particularly triclosan and essential oils such as thymol. Water soluble antimicrobials include quaternary ammonium salts such as cetyl pyridinium chloride. Enzymes are another type of active that may be used in the present compositions. Useful enzymes include those that belong to the category of proteases, lytic enzymes, plaque matrix inhibitors and oxidases. The oxidases also have whitening/cleaning activity, in addition to anti-microbial properties. Such agents are disclosed in U.S. Patent Nos. 2,946,725, and 4,051,234.

Flavouring and sweetening agents are preferably also included in the present composition. Suitable flavouring agents and sweetening agents are well known in the art. Suitable flavour levels in the present oral compositions herein are from 0.1% to 5.0%, more preferably from 0.5% to 1.5%, by weight. Typically, a flavour oil will be manufactured in a separate step and will comprise multiple components, natural and/or synthetic in origin, in order to provide a balanced flavour which is acceptable to a broad range of people. Flavour components can be selected from mint, spice, fruit, citrus, herbal, medicinal, and common food flavour types (e.g. chocolate). Illustrative, but non-limiting examples of such components include hydrocarbons such as limonene, caryophyllene, myrcene, and humulene; alcohols such as menthol, linalool, 3-decanol, and pinocarveol; ketones such as piperitone, menthone, spicatone, and 1-carvone; aldehydes such as acetaldehyde, 3-hexanal, or n-octanal; oxides such as menthofuran, piperitone oxide, or carvyl acetate-7,7 oxide; acids such as acetic and ocenoic; and sulphides such as dimethyl sulphide. Components also include esters such as menthyl acetate, benzyl isobutyrate, and 3-octyl acetate. The flavour components may also include essential oils such as peppermint oils from e.g., Mentha piperita and Mentha arvensis; spearmint oils such as those from Mentha cardiaca and Mentha spicata; sage oil, parsley oil, marjoram oil, cassia oil, clove bud oil, cinnamon oil, orange oil, , lime oil, eucalyptus oil and anise oil. Other suitable components are cinnamic aldehyde, eugenol, ionone, anethole, eucalyptol, thymol, methyl salicylate, vanillin, ethyl vanillin, and vanilla extracts. Flavour components are described in more detail in Fenaroli's Handbook of Flavor Ingredients, Third Edition, Volumes 1 & 2, CRC Press, Inc. (1995), and Steffen Arctander's Perfume and Flavour Chemicals, Volumes 1 & 2, (1969). A physiological cooling agent can also be incorporated into the flavour oil. The coolant can be any of a wide variety of materials. Included among such materials are carboxamides, menthol, acetals, ketals, diols, and mixtures thereof. Preferred coolants herein include the p-menthane carboxamide agents such as N-ethyl-p-menthane-3-carboxamide, (known commercially as "WS-3") and mixtures thereof and menthone glycerine acetal (known commercially as "MGA"). Further coolants suitable for the present invention are disclosed in WO 97/06695.

The compositions herein can further include herbal ingredients such as extracts of chamomile, oak bark, melissa, rosemary and salvia. These, and some of the herb-derived flavouring components mentioned above (such as thymol) can be included at levels just sufficient to provide a contribution to the flavour or they can be added at higher levels, such as 1% or more, in order to provide a greater therapeutic effect.

Sweetening agents which can be used include sucrose, glucose, saccharin, sucralose, dextrose, levulose, lactose, mannitol, sorbitol, fructose, maltose, xylitol, saccharin salts, thaumatin, aspartame, D-tryptophan, dihydrochalcones, acesulfame and cyclamate salts, especially sodium cyclamate, sucralose and sodium saccharin, and mixtures thereof. A composition preferably contains from 0.1 % to 3% of these agents, more preferably from 0.1 % to 1%.

The compositions may further include usual pigments, dyes and opacifiers, such as titanium dioxide. It will be appreciated that selected components for the compositions must be chemically and physically compatible with one another.

### Examples

The following examples further describe and demonstrate toothpaste embodiments within the scope of the present invention. These examples are given solely for the purpose of illustration and are not to be construed as limitations of the present invention as many variations thereof are possible.

Toothpaste compositions according to the present invention are shown below with amounts of components in weight %. These compositions are made using conventional methods.

| Ingredient | Reference | A | B | C | D | E | F | G | H | I |
|---|---|---|---|---|---|---|---|---|---|---|
| Sorbitol sol. (70%) | 37.000 | 37.000 | 37.000 | 37.000 | 37.000 | 37.00 0 | 37.000 | 37.000 | 37.000 | 37.00 0 |
| Phytic acid (50% soln) | 0.800 | 0.800 | 0.800 | 1.200 | 0.800 | 0.800 | 0.800 | 0.800 | 0.800 | 0.800 |
| Zinc Cxide | - | - | - | - | - | - | - | - | - | 0.213 |
| Citric Acid monohydrate | - | - | - | - | - | - | - | - | - | 0.365 |
| Zinc citrate | 0.533 | 0.533 | 0.533 | 0.533 | - | 0.533 | 0.533 | 0.533 | 0.533 | 0.533 |
| Potassium nitrate | 5.000 | 5.000 | 5.000 | 5.000 | 5.000 | 5.000 | | | 5.000 | 5.000 |
| Stannous fluoride | - | - | 0.454 | - | - | - | - | - | - | - |
| Sodium fluoride | 0.321 | 0.321 | - | 0.321 | 0.321 | 0.243 | 0.243 | 0.243 | 0.243 | 0.243 |
| Potassium gluconate | - | - | - | - | - | 3.300 | - | - | - | 3.664 |
| Potassium chloride | - | - | - | - | - | - | 3.690 | - | - | - |
| Potassium citrate | - | - | - | - | - | - | - | 5.05 | - | - |
| Sodium gluconate | 1.064 | 1.864 | 1.864 | 3.364 | 3.364 | - | 3.364 | 3.364 | 1.864 | - |
| Stannous chloride | 1.160 | 1.160 | 0.506 | 1.160 | 1.160 | 0.506 | 0.506 | 0.506 | 0.506 | 1.160 |
| Gantrez® S-97* | - | - | - | - | - | - | - | - | 2.000 | - |
| HEC | 0.300 | 0.300 | 0.300 | 0.300 | 0.300 | 0.300 | 0.300 | 0.300 | 0.300 | 0.300 |
| Na CMC | 1.300 | 1.300 | 1.300 | 1.300 | 1.300 | 1.300 | 1.300 | 1.300 | 1.300 | 1.300 |
| Carrageenan | 0.700 | 0.700 | 0.700 | 0.700 | 0.700 | 0.700 | 0.700 | 0.700 | 0.700 | 0.700 |
| Silica abrasive | 15.000 | 15.000 | 15.000 | 15.000 | 15.00 | 15.00 0 | 15.00 0 | 15.000 | 15.000 | 15.00 0 |
| TiO₂ (Anatase) | 0.525 | 0.525 | 0.525 | 0.525 | 0.525 | 0.525 | 0.525 | 0.525 | 0.525 | 0.525 |
| SLS (28% soln.) | 5.000 | - | - | - | - | - | - | - | - | - |
| Cocoamidopropyl Betaine | - | 3.000 | 3.000 | 3.000 | 3.000 | 3.000 | 3.000 | 3.000 | 3.000 | 3.000 |
| Na Saccharin | 0.300 | 0.300 | 0.300 | 0.300 | 0.300 | 0.300 | 0.300 | 0.300 | 0.300 | 0.300 |
| Flavor | 0.700 | 0.700 | 0.700 | 0.700 | 0.700 | 1.000 | 1.000 | 1.000 | 1.000 | 1.000 |
| KOH (50%) | - | - | - | - | - | - | - | - | - | 1.800 |
| NaOH 32% | 1.500 | 1.500 | 1.500 | 1.500 | 1.500 | 1.500 | 1.500 | 1.500 | 1.500 | - |
| Water and minors, e.g., color soln. | qs | qs | qs | qs | qs | qs | qs | qs | qs | qs |
| | | | | | | | | | | |
| Target pH | 6.0 | 6.0 | 6.0 | 6.0 | 6.0 | 6.0 | 6.0 | 6.0 | 6.0 | 6.0 |

| | | | | | | | | | | |
|---|---|---|---|---|---|---|---|---|---|---|
| * Methylvinylether/maleic acid copolymer | | | | | | | | | | |

The soluble fluoride levels of the reference sample and examples A and B were measured using the method outlined above and were found to be 220 ppm, 330 ppm and 325 ppm respectively, in the 1:3 diluted composition.

The dimensions and values disclosed herein are not to be understood as being strictly limited to the exact numerical values recited. Instead, unless otherwise specified, each such dimension is intended to mean both the recited value and a functionally equivalent range surrounding that value. For example, a dimension disclosed as "40 mm" is intended to mean "about 40 mm".

## Claims

1. A single phase oral care composition comprising:
a. a stannous salt delivering a stannous ion;
b. a potassium salt delivering a potassium ion;
c. a chelant;
d. a fluoride ion source;
e. less than 0.01 % of an alkyl sulphate or an alkyl ethoxylate sulphate; and
f. a surfactant selected from a cationic, an amphoteric surfactant, a nonionic surfactant or mixtures thereof;
wherein the oral care composition provides a soluble fluoride ion level of greater than 50% of the total fluoride ion.

2. A composition according to Claim 1 wherein the oral care composition comprises less than 0.01 % sodium lauryl sulphate.

3. A composition according to any preceding claim wherein the fluoride ion source is selected from sodium fluoride, potassium fluoride, stannous fluoride and mixtures thereof.

4. A composition according to any preceding claim which provides a soluble fluoride ion level greater than 75% of the total fluoride ion.

5. A composition according to any preceding claim wherein the molar ratio of the chelant to the stannous ion delivered from the stannous salt is at least 0.70:1 and wherein the chelant has a molecular weight of less than 1000.

6. A composition according to any preceding claim wherein the percentage weight ratio of the chelant to the stannous ion delivered from the stannous salt is at least 2:1 and wherein the chelant has a molecular weight of greater than 1000.

7. A composition according to any preceding claim which further comprises a surfactant selected from an amphoteric surfactant, a nonionic surfactant and mixtures thereof

8. A composition according to any preceding claim which further comprises a surfactant selected from cocoamidoethyl betaine; cocamidopropyl betaine; lauramidopropyl betaine; lauryl betaine and mixtures there of.

9. A composition according to any preceding claim wherein the stannous salt is selected from stannous chloride, stannous fluoride, stannous gluconate and mixtures thereof.

10. A composition according to any preceding claim wherein the potassium salt is selected from potassium nitrate, potassium gluconate, potassium citrate and mixtures thereof.

11. A composition according to any preceding claim wherein the oral care composition additionally comprises potassium hydroxide.

12. A composition according to any preceding claim which further comprises a source of zinc ions.

13. A composition according to Claim 12 wherein the zinc ions are provided from zinc citrate, zinc gluconate, zinc lactate and mixtures thereof.

14. A composition according to any preceding claim which further comprises a silica abrasive.

15. A composition according to Claim 1 wherein the stannous salt is stannous chloride; the potassium salt is potassium nitrate and wherein the composition further comprises cocamidopropyl betaine and sodium fluoride.

16. A composition according to Claim 1 wherein the stannous salt is selected from stannous chloride, stannous fluoride and mixtures thereof; the potassium salt is potassium nitrate and wherein the composition further comprises cocamidopropyl betaine.
